# EUROPEAN PATENT SPECIFICATION

(11) **EP 1 150 123 B1**
(45) Date of publication and mention of the grant of the patent: **12.07.2006**
(21) Application number: 00109063.8
(22) Date of filing: 28.04.2000
(51) Int. Cl.: G01N 33/68, G01N 33/573

(54) **Diagnosis of liver fibrosis with serum marker algorithms**
Diagnose von Leberfibrose mit Hilfe von Serummarkeralgorithmen
Diagnose de la fibrose du foie avec un algorithme des marqueurs sériques

(43) Date of publication of application: 31.10.2001
(73) Proprietor: Bayer Aktiengesellschaft, 51368 Leverkusen (DE); BAYER CORPORATION, Pittsburgh, PA 15205-9741 (US)
(72) Inventor: Völker, Michael Dr., 51061 Köln (DE); Becka, Michael Dr., 59469 Ense (DE); Kroll, Werner Dr., 42659 Solingen (DE); Knorr, Andreas Dr., 40699 Erkrath (DE); Unger, Sylvia Dr., 69117 Heidelberg (DE); Gehrmann, Mathias Dr., 51373 Leverkusen (DE); Hennig, Guido Dr., 47798 Krefeld (DE); Burchardt, Elmar-Reinhold, 42113 Wuppertal (DE)
(74) Representative: Petrovicki, Wolfgang

(56) References cited:
- EP-A- 0 911 343
- CHEMICAL ABSTRACTS, vol. 130, no. 7, 15 February 1999 (1999-02-15) Columbus, Ohio, US; abstract no. 78389, C. PILETTE ET AL.: "Histopathological evaluation of liver fibrosis: quantitative image analysis vs. semi-quantitative scores: comparison with serum markers." page 332; column 1; XP002149460 & J. HEPATOL., vol. 28, no. 3, 1998, pages 439-446,
- J. P. TEARE ET AL.: "Comparison of serum procollagen III peptide concentrations and PGA index for assessment of hepatic fibrosis" LANCET THE., vol. 342, no. 8876, 9 October 1993 (1993-10-09), pages 895-898, XP002149457 LANCET LIMITED. LONDON., GB ISSN: 0140-6736
- J. KROPF ET AL.: "Efficacy of serum laminin measurement for diagnosis of fibrotic liver diseases" CLINICAL CHEMISTRY, vol. 34, no. 10, October 1988 (1988-10), pages 2026-2030, XP002149458 WINSTON US
- J. GU¹CHOT ET AL.: "Diagnostic accuracy of hyaluronan and type III procollagen amino-terminal peptide serum assays as markers of liver fibrosis in chronic viral hepatitis C evaluated by ROC curve analysis." CLINICAL CHEMISTRY, vol. 42, no. 4, April 1996 (1996-04), pages 558-563, XP002149459 WINSTON US
- CHEMICAL ABSTRACTS, vol. 131, no. 24, 13 December 1999 (1999-12-13) Columbus, Ohio, US; abstract no. 321003, R. ENDO ET AL.: "Serial changes in the serum levels of type IV collagen, matrix metalloproteinase-2 and tissue inhibitor of metalloproteinase-2 in patients with acute and fulminant hepatitis" page 496; column 2; XP002149461 & IWATE IGAKU ZASSHI, vol. 51, no. 4, 1999, pages 371-379,
- CHEMICAL ABSTRACTS, vol. 127, no. 20, 17 November 1997 (1997-11-17) Columbus, Ohio, US; abstract no. 276418, X. JI ET AL.: "Clinical significance of serum 7S collagen and type VI collagen levels for the diagnosis of hepatic fibrosis" page 510; column 1; XP002149462 & CHIN. MED. J. (BEIJING, ENGL. ED.), vol. 110, no. 3, 1997, pages 198-201,
- CHEMICAL ABSTRACTS, vol. 131, no. 15, 11 October 1999 (1999-10-11) Columbus, Ohio, US; abstract no. 197705, M. J. P. ARTHUR ET AL.: "Tissue inhibitors of metalloproteinases: role in liver fibrosis and alcoholic liver disease" page 523; column 1; XP002149463 & ALCOHOL,:CLIN. EXP. RES., vol. 23, no. 5, 1999, pages 940-943,
- CHEMICAL ABSTRACTS, vol. 131, no. 23, 6 December 1999 (1999-12-06) Columbus, Ohio, US; abstract no. 309328, D. SCHUPPAN ET AL.: "Serum marker of liver disease." page 349; column 2; XP002149464 & DTSCH. MED. WOCHENSCHR., vol. 124, no. 41, 1999, pages 1213-1218,
- CHEMICAL ABSTRACTS, vol. 130, no. 16, 19 April 1999 (1999-04-19) Columbus, Ohio, US; abstract no. 208278, A. E. KOSSAKOWSKA ET AL.: "Altered balance between matrix metalloproteinases and their inhibitors in experimental biliary fibrosis" page 450; column 2; XP002149465 & AM. J. PATHOL., vol. 153, no. 6, 1998, pages 1895-1902,

## Description

Progressive fibrotic disease of the liver are a major cause of morbidity and mortality throughout the world. Recent scientific advances demonstrate that the pathogenic process of fibrosis in liver is critically dependent on proliferation and activation of hepatic stellate cells (also called lipocytes, fat-storing or Ito cells) which synthesize and secrete excess extracellular matrix proteins (1). Moreover it is evident that this process is common to liver disease of all aetiologies. Of particular importance are chronic viral hepatitis B and C and alcoholic liver disease as well as autoimmune and genetic liver diseases, all of which lead to clinical problems via the common final pathway of progressive liver fibrosis, with the eventual development of cirrhosis.

An important concept is the distinction between hepatic fibrosis and cirrhosis. Hepatic fibrosis is a reversible accumulation of extracellular matrix in response to chronic injury in which nodules have not yet developed, whereas cirrhosis implies an irreversible process, in which thick bands of matrix fully encircle the parenchyma, forming nodules. Consequently, any therapy must be directed towards patients with reversible disease (fibrosis), which will require early identification and monitoring of those at risk (2).

Severity and progression of liver fibrosis are difficult to assess, with liver biopsy currently remaining the most reliable clinical method. The qualitative evaluation of hepatic fibrosis by biopsies is limited by interobserver variability. Biopsies are clearly inadequate for the early clinical phase of drug development, where there is an imperative to employ less invasive methods that identify effective compounds within a commercially acceptable time frame, usually measured in weeks to a maximum of three months of experimental therapeutic exposure. Further disadvantages are the low diagnostic specificity and the risk of bleeding. Therefore there is a need for surrogate markers of liver fibrosis. Serum tests allow a non-invasive assessment of of fibrogenesis and fibrolysis in the liver and can be done repeatedly and at short time intervals (3). Serum tests measuring the dynamic processes of extracellular matrix synthesis (fibrogenesis) and extracellular matrix degradation (fibrolysis) reflect the amount of extracellular matrix present, the degree of fibrosis or the ongoing process of architectural change of the liver (4).

The current state of the art in measuring surrogate of liver fibrosis is poorly developed. Previous studies have suggested that serum levels of extracellular matrix proteins (or their cleavage fragments) may be used to assess the severity and progression of liver fibrosis (4,5). Different serum markers have been investigated and correlations with liver biopsies and and severity of liver diseases have been found (6).

Some of the makers used for the assessment of liver fibrosis are PIIINP, Laminin, Hyaluronan, Collagen IV, Collagen VI, Collagen XIV (Undulin), TIMP-1, Tenascin, MMP-2 and MMP-9/TIMP-1 complex (11-13). Markers are measured and their capability to assess liver fibrosis has been shown. Nevertheless, the diagnostic values of each single marker is not accurate enough to detect patients with fibrosis or cirrhosis.

The simple biological index PGA combining prothrombin time (PT), serum gamma-glutamyl transpeptidase (GGT) and apolipoprotein Al (Apo Al) and the index PGAA which includes alpha-2-macroglobulin (A₂M) to the PGA index have been described for the diagnosis of alcoholic liver disease in drinkers (7,8). Although the PGA and PGAA index have been combined with single serum marker (9, 10) serum markers have not been used for establishing algorithms for the assessment of liver diseases.

In this invention serum markers of the extracellular matrix are assembled together in a panel leading to a set of markers whose measurement will enable the calculation of an algorithm and the use of such a derived algorithm for the prediction of liver fibrosis histological score. For this purpose discriminant function analysis is used to determine which variables discriminate between the different fibrosis scores.

Generally, all new techniques have to be validated against existing standard techniques, provided there are any. The current "gold-standard" to assess fibrosis in the liver is the liver-biopsy. With the biopsy, some randomly taken tissue out of the liver is cut into slices which become examined by an expert using a microscope. There are a lot of problems associated with liver biopsies inducing some uncertainty: distribution of fibrosis in the liver (clustered fibrosis and the needle might have hit regions of the liver not affected by fibrosis), failed sample preparation (e.g. not enough tissue material), and pathologist's individuality and preferences (individual assessments). Furthermore, the fibrotic state of the liver is usually described using scores and there are a lot of different, possibly incompatible scoring systems (e.g. Scheuer Score, Ishak Scores, etc.).

For example, in a study with 24 patients, two independent pathologists had to score the same biopsy samples for each patient at two different time-points using two different scoring systems. The number of assessments where the two pathologists came to identical results ranged from only 36% to 46%.

The new technique is based on measuring serum parameters, which are directly associated with fibrotic processes, and combining them on a mathematical level yielding a fixed assessment procedure.

In order to validate the new technique the "gold-standard" is not the best but the only mean, since a priori both methods do not investigate comparable endpoints: whereas the serum parameters characterize dynamic processes, the biopsy characterizes the fibrotic manifestation at a fixed time-point. There may be a highly active fibrotic process in the liver although fibrotic tissue has not yet been developed. In contrast, there may be large clusters of fibrotic tissue in the liver but the fibrotic activity stopped or discontinued temporarily.

Although, some mathematical functions of serum parameters yielded statistically significant different mean values in different biopsy score stages. Discriminant analyses using the "gold-standard" were chosen in order to investigate the diagnostic power of those mathematical functions of serum parameters.

### Description of the immunoassays

The markers N-terminal procollagen III propeptide (PIIINP), Collagen IV, Collagen VI, Collagen XIV, Tenascin, Laminin, Hyaluronan, MMP-2, TIMP-1 and MMP-9/TIMP-1 complex are used for algorithms.

The markers are measured by making use of immunoassays. The immunoassays of the invention comprises reaction of two antibodies with a human fluid samples, wherein the capture antibody specifically binds to one epitope of the marker. The second antibody of different epitope specificity is used to detect this complex. Preferably the antibodies are monoclonal antibodies and both antibodies of said two antibodies of the assay specifically bind to the protein.

Antibody or other similar term used herein includes a whole immunoglobulin either monoclonal or polyclonal as well as antigenic fragments or immunoreactive fragments which specifically bind to the marker, including Fab, Fab', F(ab')₂ and F(v). Antibody includes also binding-proteins, especially Hyaluronic acid-binding protein (HABP).

The human fluid sample used in the assays of the invention can be any sample that contain the markers, e.g. blood, urine, sputum or broncho alveolar lavage (BAL). Typically a serum or plasma sample is employed.

Antibodies of the invention can be prepared by techniques generally known in the art, and are typically generated to a sample of the markers.

The second antibody is conjugated to a detector group, e.g. alkaline phosphatase or a fluorescence dye. Conjugates are prepared by techniques generally known in the art.

Concentration of the markers in human fluids are measured and algorithms calculated to assess the degree of fibrosis.

### Discription of algorithms

Discriminant function analysis is used to determine which variables discriminate between two or more naturally occurring groups. Computationally, it is very similar to analysis of variance. The basic idea underlying discriminant function analysis is to determine whether groups differ with regard to the mean of a variable, and then to use that variable to predict group membership (e.g., of new cases). Stated in this manner, the discriminant function problem can be rephrased as a one-way analysis of variance (ANOVA) problem. Specifically, one can ask whether or not two or more groups are significantly different from each other with respect to the mean of a particular variable. If the means for a variable are significantly different in different groups, then we can say that this variable discriminates between the groups. In the case of a single variable, the final significance test of whether or not a variable discriminates between groups is the *F* test. *F* is essentially computed as the ratio of the between-groups variance in the data over the pooled (average) within-group variance. If the between-group variance is significantly larger then there must be significant differences between means.

Usually, one includes several variables in a study in order to see which one(s) contribute to the discrimination between groups. In that case, we have a matrix of total variances and co-variances; likewise, we have a matrix of pooled within-group variances and co-variances. We can compare those two matrices via multivariate F tests in order to determined whether or not there are any significant differences (with regard to all variables) between groups. This procedure is identical to multivariate analysis of variance or MANOVA. As in MANOVA, one could first perform the multivariate test, and, if statistically significant, proceed to see which of the variables have significantly different means across the groups. Thus, even though the computations with multiple variables are more complex, the principal reasoning still applies, namely, that we are looking for variables that discriminate between groups, as evident in observed mean differences.

For a set of observations containing one or more quantitative variables and a classification variable defining groups of observations, the discrimination procedure develops a discriminant criterion to classify each observation into one of the groups. Post hoc predicting of what has happened in the past is not that difficult. It is not uncommon to obtain very good classification if one uses the same cases from which the discriminant criterion was computed. In order to get an idea of how well the current discriminant criterion "performs", one must classify (a priori) different cases, that is, cases that were not used to estimate the discriminant criterion. Only the classification of new cases allows us to assess the predictive validity of the discriminant criterion. In order to validate the derived criterion, the classification can be applied to other data sets. The data set used to derive the discriminant criterion is called the training or calibration data set.

The discriminant criterion (function(s) or algorithm), is determined by a measure of generalized squared distance. It can be based on the pooled co-variance matrix yielding a linear function. Either Mahalanobis or Euclidean distance can be used to determine proximity.

For the development of a discriminant algorithm, data of 154 subjects of an observational liver fibrosis of study were analyzed. Liver fibrosis scoring systems under view were
- the Scheuer Score (0-4),
- the Modified Ishak Score (HAI) A - Interface Hepatitis (0-4),
- the Modified Ishak Score (HAI) B - Confluent Necrosis (0-6),
- the Modified Ishak Score (HAI) C - Spotty Necrosis (0-4),
- the Modified Ishak Score (HAI) D - Portal Inflammation (0-4),
- the Modified Ishak Score (HAI) - Stage (0-6).

Applying a stepwise discriminant analysis, the following functions of serum parameters showed to have major impact on the corresponding scoring type.

| *Scoring Type* | *Surrogate Parameters* | | |
|---|---|---|---|
| **Scheurer Score**: | *ln(TIMP-1)* | *In(Collagen VI*/ *Hyaluronan)* | *ln(Hyaluronan* / *Laminin)* |
| **Modified Ishak Score A - Interface Hepatitis**: | *ln(TIMP-1)* | *In(Collagen VI*/ *Hyaluronan)* | *ln(Collagen VI*/ *Tenascin)* |
| **Modified Ishak Score B** - **Confluent Necrosis**: | *In(Hyaluronan)* | *In(Collagen VI*/ *MMP-2)* | |
| **Modified Ishak Score C** - **Spotty Necrosis**: | *In (Hyaluronan)* | *In(MMP-9_TIMP-I*/ *Tenascin)* | |
| **Modified Ishak Score D - Portal Inflammation**: | *In(Laminin)* | *In(Collagen VI*/ *TIMP-1)* | |
| **Modified Ishak Score - Stage**: | *In(TIMP-1)* | *In(Collagen VI*/*In Hyaluronan)* | *(Hyaluronan* / *Laminin)* |

A corresponding discriminant analysis yielded the following linear discriminating functions:
Scheuer Score:
   0 : -108.861 +0.283×LN(COLLAGEN VI/HYALURON) - 1.050×LN(HYALURON/LAMININ)+35.372×LN(TIMP-1)
   1 : -114.231+0.195×LN(COLLAGEN VI/HY ALURON)-0.654×LN(HYALURONILAMININ)+36.158×LN(TIMP-1)
   2 : -120649 - 0.998×LN(COLLAGEN VI/HYALURON)-2.102×LN(HYALURON/LAMININ)+36.925×LN(TIMP-1)
   3 : -123.672-1.281×LN(COLLAGEN VI/HYALURON) - 1.344×LN(HYALURON/LAMININ) 37.163×LN(TIMP-1)
   4 : -133.207 - 2.186×LN(COLLAGEN VI/HYALURON) - 1.602×LN(HY ALURON/LAMININ) +38.188×LN(TIMP-1)
Modified HAI-Stage:
   0 : -107.752-0.347×LN(COLLAGEN VI/HYALURON) - 1.493×LN(HYALURON/LAMININ) + 34.879×LN(TIMP-1)
   1 :-112.550-0.301×LN(COLLAGEN VI/HYALURON) - 1.086×LN(HYALURON/LAMININ) + 35.617×LN(TIMP-1)
   2 : -114.626-0.760×LN(COLLAGEN VI/HYALURON) - 1.270×LN(HYALURON/LAMININ) + 35.819×LN(TIMP-1)
   3 : -121.339-2 065×LN(COLLAGEN VI/HYALURON) - 2.910×LN(HYALURON/LAMININ) + 36.593×LN(TIMP-1)
   4 : -119.289-1.009×LN(COLLAGEN VI/HYALURON) - 1.271×LN(HYALURON/LAMININ) + 36.449×LN(TIMP-1)
   5 : -125.551-2.966×LN (COLLAGEN VIIHYALURON) - 2.536×LN(HYALURON/LAMININ) + 36.797×LN(TIMP-1)
   6 : -133.055-3.256×LN(COLLAGEN VI/HYALURON) - 2.329×LN(HYALURON/LAMININ) + 37.695×LN(TIMP-1)

Discriminating functions used herein also includes other combinations of markers from the list of N-terminal procollagen III propeptide (PIIINP), Collagen IV, Collagen VI, Collagen XIV, Tenascin, Laminin, Hyaluronan, MMP-2, TIMP-1 and MMP-9/TIMP-1 complex and also factors between 0 and 200.

Different scores need different algorithm form the list of markers and factors.

Validating the established discriminant algorithm, data of 222 separate subjetcs of a liver fibrosis study were classified regarding the Scheuer Score and the HAI-Stage. Overall rate of agreement for the calibration data set was 37% for the Scheuer Score (κ = *0.18,* range for individual scores: 28% - 69%) and 28% for the HAI-Stage (κ = *0.14,* range for individual scores: 3% - 62%). Details of rates of agreement are given below.

| Type | Score | No. of assessments | No. of agreements | % |
|---|---|---|---|---|
| Scheuer Score | [0] | 53 | 16 | 30.2 |
| | [1] | 86 | 31 | 36.0 |
| | [2] | 25 | 7 | 28.0 |
| | [3] | 32 | 9 | 28.1 |
| | [4] | 26 | 18 | 69.2 |
| | all | 222 | 81 | 36.5 |
| modified HAI-Stage | [0] | 53 | 15 | 28.3 |
| | [1] | 57 | 14 | 24.6 |
| | [2] | 31 | 1 | 3.2 |
| | [3] | 22 | 6 | 27.3 |
| | [4] | 23 | 6 | 26.1 |
| | [5] | 10 | 3 | 30.0 |
| | [6] | 26 | 16 | 61.5 |
| | all | 222 | 61 | 27.5 |

| | | | | | | | | |
|---|---|---|---|---|---|---|---|---|
| | | | | | | 95% | 95% | |
| | | | | | | Lower | Upper | P (kappa=0) |
| | | | | | | CI | CI | asympt. |
| Type | | P(A) | P(C) | kappa | ASE | border | border | 2-sided |
| Scheuer Score | simple kappa | 0.365 | 0.223 | 0.183 | 0.042 | 0.101 | 0.265 | < 0.0001 |
| | weighted kappa | | | 0.381 | 0.045 | 0.292 | 0.469 | < 0.0001 |
| Modified HAI | simple kappa | 0.275 | 0.158 | 0.138 | 0.036 | 0.069 | 0.208 | < 0.0001 |
| | weighted kappa | | | 0.361 | 0.044 | 0.274 | 0.447 | < 0.0001 |

Considering the more relevant dichotomous outcomes no/mild: Score 0-2 (Scheuer Score), Score 0-3 (HAI-Stage) and moderate/severe: Score 3-4 (Scheuer Score), Score 4-6 (HAI-Stage), the results were completely comparable with the results of the calibration data. The overall rate of agreement for the validation set was 79% for the Scheuer Score (κ = *0.46,* no/mild 85%, moderate/severe: 62%) and 76% for the HAI-Stage (κ = *0.40,* no/mild 80%, moderate/severe: 63%). Using biopsy results, about 26% of the 222 cases under view were assessed as moderate/severe by the central pathologist yielding unbalanced group sizes. Since percentages strongly depend on the actual group size, the observed smaller rate of agreement for the moderate/severe group may be due to this fact. Details are given below.

| Type | Score | No. of assessments | No. of agreements | % |
|---|---|---|---|---|
| Scheuer Score | [0-2] | 164 | 140 | 85.4 |
| | [3-4] | 58 | 36 | 62.1 |
| | all | 222 | 176 | 79.3 |
| modified HAI-Stage | [0-3] | 163 | 131 | 80.4 |
| | [4-6] | 59 | 37 | 62.7 |
| | all | 222 | 168 | 75.7 |

| | | | | | | | | |
|---|---|---|---|---|---|---|---|---|
| | | | | | 95% | 95% | | |
| | | | | | lower | upper | P(kappa=0) | P(kappa=0) |
| | | | | | Cl | Cl | asympt. | exact |
| Type | P(A) | P(C) | Kappa | ASE | border | border | 2-sided | 2-sided |
| Scheuer Score Simple Kappa | 0.793 | 0.610 | 0.469 | 0.067 | 0.338 | 0.600 | < 0.0001 | < 0.0001 |
| Modified Simple Kappa | 0.757 | 0.589 | 0.409 | 0.067 | 0.278 | 0.539 | < 0.0001 | < 0.0001 |
| HAI-Stage | | | | | | | | |

### Literature

1. Friedman SL
   The cellular basis of hepatic fibrosis:Mechanism and treatment strategies. N Engl J Med 1993; 328: 1828-1835
2. Friedman SL
   Molecular mechanism of hepatic fibrosis and principle of therapy J Gastroenterol 1997; 32: 424-430
3. Hayasaka A, Saisho H
   Serum markers as tools to monitor liver fibrosis Digestion 1998; 59: 381-384
4. Schuppan D, Stolzel U, Oesterling C, Somasundaram R
   Serum assays for liver fibrosis. J Hepatol 1995; 22 (Suppl 2): 82-88
5. Murawaki Y, Ikuta Y, Nishimura Y, Koda M, Kawasaki H
   Serum markers for connective tissue turnover in patients with chronic hepatitis C: A comparative analysis. J Hepatol 1995; 23: 145-152
6. Wong VS, Hughes V, Trull A, Wight DGD, Petrik J, Alexander GJM Serum hyaluronic acid is a useful marker of liver fibrosis in chronic hepatitis C virus infection
   J Viral Hepatitis 1998; 5: 187-192
7. Poynard T, Aubert A, Bedossa P, Abella A, Naveau S, Paraf F, Chapu JC
   A simple biological index for detection of alcoholic liver disease in drinkers Gastroenterology 1991; 100: 1397-1402
8. Naveau S, Poynard T, Benattat C, Bedossa P, Chaput JC
   Alpha-2 macroglobulin and hepatic fibrosis:diagnostic interest
   Dig Dis Sci 1994; 11: 2426-2432
9. Oberti F, Valsesia E, Pilette C, Rousselet MC, Bedossa P, Aube C, Gallois Y, Rifflet H, Maiga MY, Penneau-Fontbonne D, Cales P
   Noninvasive diagnosis of hepatic fibrosis and cirrhosis
   Gastroenterology 1997; 113: 1609-1616
10. Teare JP, Sherman D, Greenfield SM, Simpson J, Catterall AP, Murray-Lyon
   IM, Peters TJ, Williams R, Thompson RPH
   The Lancet 1993; 342: 895-898
11. Pilette, Ch. et al
   J. Hepatol. 1998; 28, 439-446
12. Guéchot, J. et al.
   Clin. Chem. 1996; 42, 558-563
13. Ji, X. et al.
   Chin. Med. J. 1997; 100 198-201

## Claims

1. A method for diagnosing liver fibrosis wherein two or more diagnostic markers are measured in vitro in a body fluid and the measurements are combined on a mathematic level by a mathematic algorithm **characterized in that** the diagnostic markers are selected from the group N- terminal procollagen III propeptide (PIIINP), Collagen IV, Collagen VI, Collagen XIV, Tenascin, Laminin, Hyaluronan, MMP-2, TIMP-1 and MMP-9/TIMP-1 complex.

2. A method according to claim 1 wherein body fluid is blood, serum, plasma,urine, sputum or broncho alveolar lavage.

## Patentansprüche

1. Verfahren zur Diagnose von Leberfibrose, worin zwei oder mehr diagnostische Marker in vitro in einer Körperflüssigkeit gemessen werden und die Messungen durch einen mathematischen Algorithmus auf einer mathematischen Ebene kombiniert werden, **dadurch gekennzeichnet, dass** die diagnostischen Marker aus der aus N-terminalem Procollagen-III-Propeptid (PIIINP), Collagen IV, Collagen VI, Collagen XIV, Tenascin, Laminin, Hyaluronan, MMP-2, TIMP-1 und einem MMP-9/TIMP-1-Komplex bestehenden Gruppe ausgewählt sind.

2. Verfahren nach Anspruch 1, worin die Körperflüssigkeit Blut, Serum, Plasma, Urin, Sputum oder bronchoalveoläre Lavage ist.

## Revendications

1. Procédé pour diagnostiquer la fibrose du foie, dans lequel deux marqueurs diagnostiques ou plus sont mesurés *in vitro* dans un liquide organique et les mesures sont combinées à un niveau mathématique par un algorithme mathématique **caractérisé en ce que** les marqueurs diagnostiques sont choisis dans le groupe propeptide procollagène III N-terminal (PIIINP), collagène IV, collagène VI, collagène XIV, tenascine, laminine, hyaluronane, MMP-2, TIMP-1 et complexe MMP-9/TIMP-1.

2. Procédé selon la revendication 1 dans lequel le liquide organique est du sang, du sérum, du plasma, de l'urine, du crachat ou un lavage broncho-alvéolaire.
